(19) Europäisches Patentamt
European Patent Office
Office européen des brevets

(11) Publication number: 0 048 040
B1

(12) **EUROPEAN PATENT SPECIFICATION**

(45) Date of publication of patent specification: **29.08.84**

(21) Application number: **81200861.3**

(22) Date of filing: **30.07.81**

(51) Int. Cl.$^3$: **C 07 D 307/79,**
C 07 D 413/04, A 01 N 47/24,
A 01 N 43/82

(54) **7-Substituted 2,3-dihydrobenzofurans, their preparation and their use as pesticides or as chemical intermediates.**

(30) Priority: **15.09.80 US 187234**
**15.09.80 US 187235**
**15.09.80 US 187240**

(43) Date of publication of application:
**24.03.82 Bulletin 82/12**

(45) Publication of the grant of the patent:
**29.08.84 Bulletin 84/35**

(84) Designated Contracting States:
**BE CH DE FR GB IT LI NL SE**

(56) References cited:
**GB-A-1 336 913**
**US-A-3 547 955**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Pilgram, Kurt Hans Gerhard**
**2607 Warwick Lane**
**Modesto California 95350 (US)**
Inventor: **Skiles, Richard Daniel**
**2912 Debbie Lane**
**Modesto California 95350 (US)**
Inventor: **Tieman, Charles Henry**
**2209 Fremont Drive**
**Modesto California 95350 (US)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 048 040 B1

Courier Press, Leamington Spa, England.

## Description

This invention relates to 7-substituted 2,3-dihydrobenzofurans, their preparation, and their use as pesticides or as intermediates in the preparation of pesticidally active compounds.

According to the invention there are provided 7-substituted 2,3-dihydrobenzofurans of general formula

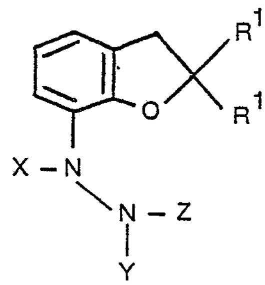

(I)

wherein each $R^1$ represents a hydrogen atom or a methyl group; X, Y and Z together represent a bridging group —CO—O—C(OR)=, or X is a chlorocarbonyl group, Y is hydrogen and Z is a carboxylate ester group —COOR, or X and Y are both hydrogen and Z is a carboxylate ester group —COOR or a sulfonic acid group —$SO_3H$, or a salt thereof; and R represents a methyl, ethyl or propynyl group.

Compounds of formula I wherein X, Y and Z together represent a bridging group —CO—O—C(OR)= or wherein X is a chlorocarbonyl group, Y is hydrogen and Z is a carboxylate ester group —COOR have been found to exhibit pesticidal, including both insecticidal and acaricidal, activity. Compounds of formula I wherein X and Y are both hydrogen and Z is a carboxylate ester group —COOR or a sulphonic acid group —$SO_3H$, or a salt, e.g. sodium or potassium salt, and wherein X is a chlorocarbonyl group, Y is hydrogen and Z is a carboxylate ester group —COOR have utility as chemical intermediates in the preparation of pesticidally-active compounds of formula I.

A preferred, pesticidally active, sub-group of compounds of formula I is that wherein when each $R^1$ represents a hydrogen atom, X, Y and Z together represent a bridging group —CO—O—C($OCH_3$)=, and when each $R^1$ represents a methyl group, X, Y and Z together represent a bridging group —CO—O—C—(OR)= wherein R represents a methyl, ethyl or propynyl group or X is a chlorocarbonyl group, Y is hydrogen and Z is a carboxymethyl or carboxyethyl group.

The compound of formula I wherein $R^1$ represents a hydrogen atom and X, Y and Z together represent the bridging group —CO—O—C($OCH_3$)= is 3-(2,3-dihydrobenzofuran-7-yl)-5-methoxy-1,3,4-oxadiazol-2(3H)-one, and has the formula

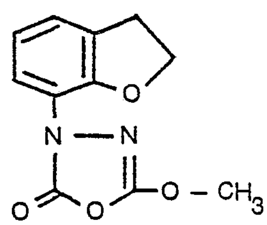

(A)

The compounds of formula I wherein $R^1$ represents a methyl group and X, Y and Z together represent the bridging group —CO—O—C(OR)= have the general formula

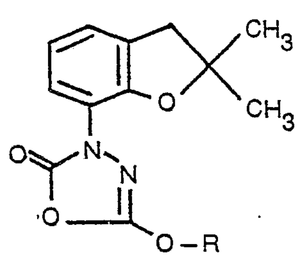

(II)

wherein R is methyl, ethyl or propynyl.

The compounds of formula I wherein $R^1$ represents a methyl group, X is a chlorocarbonyl group, Y is hydrogen and Z is a carboxymethyl or carboxyethyl group have the general formula

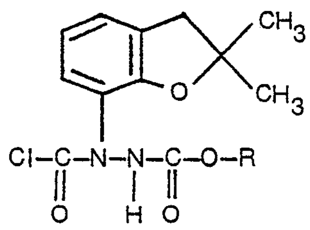

(III)

wherein R is methyl or ethyl.

In accordance with the invention, compounds of formula I wherein X, Y and Z together represent a bridging group —CO—O—C(OR)=, or X is a chlorocarbonyl group, Y is hydrogen and Z is a carboxylate ester group —COOR, and R represents a methyl, ethyl or propynyl group, may be prepared by a process which comprises reacting an ester of formula

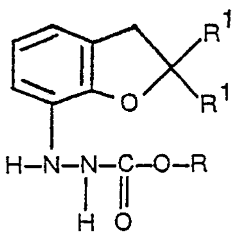

(IV)

wherein R and $R^1$ are as defined above with phosgene in an inert solvent, followed where necessary by treating the resulting compound of formula I wherein X is a chlorocarbonyl group, Y is hydrogen and Z is a carboxylate ester group —COOR with a base to produce a compound of formula I wherein X, Y and Z together represent a bridging group —CO—O—C(OR)=.

Reaction of the ester of formula IV with phosgene is effected in an inert solvent, such as benzene, conveniently at ambient temperature. Treatment of the resulting compound of formula I wherein X is a chlorocarbonyl group, Y is hydrogen and Z is a carboxylate ester group with a base may conveniently be effected in the presence of a liquid alkanol, such as methanol, as solvent and at a temperature ranging from ambient temperature to the reflux temperature of the reaction mixture. Suitable bases include tertiary amine bases. Ethyldiisopropylamine and triethylamine are examples of preferred bases.

The esters of formula IV, which are themselves compounds of formula I wherein X and Y are both hydrogen and Z is a carboxylate ester group —COOR, may be prepared by reacting 2,3-dihydro-2,2-dimethyl-7-hydrazinobenzofuran or 2,3-dihydro-7-hydrazinobenzofuran, or an acid-addition salt thereof, with a chloroformate ester of formula

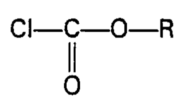

(V)

where R is as defined above in an inert solvent, such as tetrahydrofuran, in the presence of a base, for example a tertiary amine such as triethylamine or ethyldiisopropylamine.

2,3-Dihydro-2,2-dimethyl-7-hydrazinobenzofuran and 2,3-dihydro-7-hydrazinobenzofuran, and their acid-addition salts, may be prepared, via (2,3-dihydro-2,2-dimethylbenzofuran-7-yl)hydrazinesulfonic acid and (2,3-dihydrobenzofuran-7-yl)hydrazine-sulfonic acid, or salts thereof (e.g. sodium or potassium salts), from 2,3-dihydro-2,2-dimethyl-7-nitrobenzofuran and 2,3-dihydro-7-nitrobenzofuran respectively, as described in the examples hereinafter. (2,3-Dihydro-2,2-dimethylbenzofuran-7yl)hydrazine-sulfonic acid, (2,3-dihydrobenzofuran-7-yl)hydrazinesulfonic acid and their salts are compounds of formula I wherein X and Y are both hydrogen and Z is a sulfonic acid group —$SO_3H$, and salts thereof.

The invention also comprehends a pesticidal composition comprising a 2,3-dihydrobenzofuran of formula I wherein each $R^1$ is hydrogen and X, Y and Z together represent a bridging group —CO—O—C($OCH_3$)= or wherein each $R^1$ is methyl and X, Y and Z together represent a bridging group —CO—OC(OR)=, wherein R represents a methyl, ethyl or propynyl group, or X is a chlorocarbonyl group, Y is hydrogen and Z is a carboxymethyl or carboxyethyl group, in association with an inert carrier therefor. Such a composition is prepared by bringing the compound of formula I into association with the inert carrier. The invention also provides a method of combating pests at a locus which comprises applying to the locus such a compound of formula I or a pesticidal composition as defined above.

The term "carrier" as used herein mean a solid or fluid material, which may be inorganic or organic and of synthetic or natural origin, with which the active compound is mixed or formulated to facilitate its application to the locus to be treated, or its storage, transport or handling.

Suitable solid carriers are natural and synthetic clays and silicates, for example, natural silicas such as diatomaceous earths; magnesium silicates, for example talcs; magnesium aluminium silicates, for example, attapulgites and vermiculites; aluminium silicates, for example, kaolinites, montmorillonites and micas; calcium carbonates; calcium sulfate; synthetic hydrated silicon oxides and synthetic calcium or aluminium silicates; elements such as, for example, carbon and sulfur; natural and synthetic resins such as, for example, coumarone resins, polyvinyl chloride and styrene polymers and copolymers; solid polychlorophenols; bitumen; waxes such as, for example, beeswax, paraffin wax, and chlorinated mineral waxes; and solid fertilizers, for example, superphosphates.

Examples of suitable fluid carriers are water, alcohols, such as for example, isopropanol; glycols; ketones such as for example, acetone, methyl ethyl ketone, methyl isobutyl ketone and cyclohexanone; ethers; aromatic hydrocarbons such as for example, benzene, toluene and xylene; petroleum fractions such as for example, kerosene, light mineral oils; chlorinated hydrocarbons, such as for example, carbon tetrachloride, perchloroethylene, trichloroethane, including liquified normally vaporous gaseous compounds. Mixtures of different liquids are often suitable. The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium or calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, or sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions may be formulated as wettable powders dusts, granules, solutions, emulsifiable concentrates, emulsions, suspension concentrates and areosols. Wettable powders are usually compounded to contain 25, 50 or 75% by weight and usually contain in addition to solid carrier, 3—10% by weight of a dispersing agent, 15% of a surfaceactive agent and where necessary, 0—10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 1/2—10% by weight of toxicant. Granules are usually prepared to have a size between 10 and 100 BS mesh (1.676—0,152 mm), and may be manufactured by agglomeration or impregnation techniques. Generally, granules will contain 1/2—25% by weight toxicant and 0—1% by weight of additives such as stabilizers, slow release modifiers and binding agents. Emulsifiable concentrates usually contain, in addition to the solvent and, when necessary, cosolvent, 10—50% weight per volume toxicant, 2—20% weight per volume emulsifiers and 0—20% weight per volume of appropriate additives such as stabilizers, penetrants and corrosion inhibitors. Suspension concentrates are compounded so as to obtain a stable, non-sedimenting, flowable product and usually contain 0—75% weight toxicant, 0.5—5% weight of dispersing agents, 1—5% of surface-active agent, 0.1—10% weight of suspending agents, such as defoamers, corrosion inhibitors, stabilizers, penetrants and stickers, and as carrier, water or an organic liquid in which the toxicant is substantially insoluble; certain organic solids or inorganic salts may be dissolved in the carrier to assist in preventing sedimentation or as antifreeze agents for water.

Aqueous dispersions and emulsions, for example, compositions obtained by diluting a wettable powder or a concentrate, also are contemplated. The said emulsions may be of the water-in-oil or the oil-in-water type, and may have a thick mayonnaise-like consistency.

The compositions may also contain other ingredients, for example, other compounds possessing pesticidal, especially insectical, acaricidal, herbicidal or fungicidal properties.

The method of applying the pesticidal compound to control pests comprises applying it, ordinarily in a composition of one of the aforementioned types, to a locus to be protected from the pests, such as the foliage and/or the fruit of plants. It is of course applied in an amount sufficient to exert the desired action. This dosage is dependent upon many factos, including the carrier employed, the method and conditions of the application, whether the formulation is present at a locus in the form of an aerosol, or as a film, or as discrete particles, or the thickness of film or size of particles. In general, however, the effective dosage of the pesticidal compound at the locus to be protected — i.e., the dosage which the pests contact — is of the order of 0.001 to 0.05% based on the total weight of the formulation, though under some circumstances the effective concentrations will be as little as 0.0011% or as much as 2%, on the same basis.

The invention will be further understood from the following Examples, in which the identity of each precursor, and of each product, was confirmed by appropriate chemical and spectral analyses.

Example 1

2-(2-chlorocarbonyl)-2-(2,3-dihydro-2,2-dimethylbenzofuran-7-yl)hydrazinecarboxylic acid methyl ester (1)

3 g of a commercial 10% palladium-on-charcoal catalyst was added to a solution of 82.2 g of 2,3-dihydro-2,2-dimethyl-7-nitrobenzofuran (prepared by the method described in Unites States patent 3,412,110) in 500 ml of tetrahydrofuran. The resulting mixture was treated with hydrogen (50 p.s.i.g.) in a Parr shaker, then filtered. The solvent was evaporated from the filtrate. The residue was dissolved in one liter of anhydrous ether cooled to 0°C. The solution was treated with a slight excess of gaseous anhydrous hydrogen chloride.

The resulting mixture was filtered and the solid was dried to give 7-amino-2,3-dihydro-2,2-dimethylbenzofuran hydrochloride (*1A*), as a white solid.

A chilled (5°C) solution of 82.7 g of *1A*, 146 ml of concentrated hydrochloric acid and 450 ml of water was diazotized by the drop-by-drop addition over a 30-minute period of a solution of 31.5 g sodium nitrite in 45 ml of water. After filtration, the resulting solution was stirred at 5°C for 45 minutes and then added drop-by-drop to a stirred solution of 367 g of sodium sulfite in 705 ml of water, at 5°C. The resulting mixture was stirred for 2 hours at room temperature. Then a slurry of 72.2 g of sodium dithionite in 100 ml of water was added in portions. The resulting mixture was stirred for 2 hours at room temperature, then heated at 70°C for 10 minutes, and 500 g of potassium chloride was added. After 18 hours, the resulting mixture was cooled at 5°C and filtered. The solid was dried to give the potassium salt of (2,3-dihydro-2,2-dimethylbenzofuran-7-yl)hydrazinesulfonic acid (*1B*), as an off-white solid, m.p.: approximately 120°C (with decomposition).

18 g of hydrogen chloride was introduced into a stirred slurry of 56 g of *1B* in 250 ml of ethanol. The resulting mixture was stirred for 3 hours, and the solvent was evaporated. The residue was dissolved in 50 ml of water, the solution was made basic with aqueous sodium hydroxide, and extracted with ether. The extract was washed with cold water, dried ($MgSO_4$), and filtered, and the solvent was evaporated to give 2,3-dihydro-2,2-dimethyl-7-hydrazinobenzofuran (*1C*) as a dark oil.

17.8 g of *1C* thus prepared and 12.9 g of ethyldiisopropylamine were dissolved in 200 ml of tetrahydrofuran. 9.45 g of methyl chloroformate was added drop-by-drop to the stirred solution, and the resulting mixture was stirred for one hour. The solvent then was evaporated, the residue was washed with water, extracted with ether, and the extract was dried. Part of the ether was evaporated from the extract and the remaining solution was cooled. The resulting solid was collected to give the methyl ester of 2-(2,3-dihydro-2,2-dimethylbenzofuran-7-yl)hydrazinecarboxylic acid (*1D*), as a white solid, m.p.; 101—102°C.

A solution of 6.3 g of phosgene in 60 ml of benzene was added to a solution of 13.5 g of *1D* in 100 ml benzene at room temperature. The resulting mixture was held for 24 hours at room temperature, and the solvent was evaporated to give *1*, as a white crystalline solid, m.p.: 118—120°C.

Examples 2 and 3

By the general procedures described in Example 1: 2-(2-chlorocarbonyl)-2-(2,3-dihydro-2,2-dimethyl-benzofuran-8-yl)hydrazinecarboxylic acid ethyl ester, (*2*) was prepared, as a solid, m.p.: 113—115°C, from the ethyl ester of 2-(2,3-dihydro-2,2-dimethylbenzofuran-7-yl)hydrazinecarboxylic acid, a white solid, m.p.: 94—95°C.

2-(2-Chlorocarbonyl)-2-(2,3-dihydro-2,2-dimethylbenzofuran-7-yl)hydrazinecarboxylic acid propynyl ester, (*3*), was prepared, as an oil, from the propynyl ester of 2-(2,3-dihydro-2,2-dimethylbenzofuran-7-yl)hydrazinecarboxylic acid, a white solid, m.p. 70—80°C.

Example 4

3-(2,3-dihydro-2,2-dimethylbenzofuran-7-yl)-5-methoxy-1,3,4-oxadiazol-2(3H)-one (*4*)

A solution of 13 g of *1* and 15 g of ethyldiisopropylamine in 200 ml of methanol was refluxed for 15 minutes. After cooling, the solvent was evaporated. The residue was triturated with water, acidified with hydrochloric acid and extracted with ether. The extract was dried ($MgSO_4$) and the solvent was evaporated. The residue was crystallized from ether-hexane to give *4*, as a white solid, m.p.: 52—54°C.

Examples 5 and 6

By the general procedure described in Example 4: 3-(2,3-dihydro-2,2-dimethylbenzofuran-7-yl)-5-ethoxy-1,3,4-oxodiazol-2(3H)-one (*5*) was prepared, as an oil, from *2*.

3-(2,3-Dihydro-2,2-dimethylbenzofuran-7-yl)-5-propynyloxy-1,3,4-oxadiazol-2(3H)-one (*6*), was prepared, as a solid, m.p.: 72—74°C, from *3*.

Example 7

2-(Chlorocarbonyl)-2-(2,3-dihydrobenzofuran-7-yl)hydrazinecarboxylic acid, methyl ester (*7*)

39.4 g of 2,3-dihydro-7-nitrobenzofuran (prepared by the method described in Journal of Heterocyclic Chemistry, vol. 5, No. 1, p. 1 (1968)) in 200 ml of tetrahydrofuran was hydrogenated in a Parr shaker (50 p.s.i.g. hydrogen pressure, Raney nickel catalyst, 3 hours, room temperature). The resulting mixture was filtered and the solvent was evaporated from the filtrate. The residue was recrystallized

from ether to give 2,3-dihydro-7-benzofuranamine (*7A*), as a colourless solid, m.p.: 70—72°C.

25 g of *7A* was added to a solution of 65 ml of concentrated hydrochloric acid in 200 ml of water. The mixture was warmed to 45°C, and then stirred at room temperature for 18 hours. The mixture was chilled to 5°C during the addition (over a period of 20 minutes) of a solution of 14 g of sodium nitrite in 20 ml of water. The resulting mixture was stirred at 45°C for 45 minutes, then added drop-by-drop over a period of 15 minutes to a mixture of 164 g of sodium sulfite and 315 ml of water, at 5—10°C. The mixture was stirred at room temperature for 3 hours. A sample of the product sodium (2,3-dihydrobenzofuran-7-yl)diazinesulfonate, monohydrate, (*7B*), a yellow solid, m.p.: approximately 180°C (with decomposition), was isolated for analysis and the remaining mixture was used directly in the following step.

A solution of 32.2 g of sodium dithionite in 100 ml of water was added drop-by-drop over a period of 15 minutes, with stirring, at 25°C, to the final mixture of the preceding step. The resulting mixture was stirred for 2 hours at room temperature, then at 70°C for 10 minutes. Then, 148 g of potassium chloride was added and the mixture was stirred at room temperature for 18 hours. The resulting mixture was chilled to 5°C and filtered. The solid was dried to give potassium 2-(2,3-dihydrobenzofuran-7-yl)hydrazinesulfonate (*7C*), m.p.: 255°C (with decomposition).

A mixture of 45.3 g of *7C* and 500 ml of anhydrous methanol was chilled to 5—10°C during the addition of 23 g of anhydrous hydrochloric acid, stirred at room temperature for 18 hours, and filtered. The solvent was evaporated from the filtrate, and residue being 7-hydrazine-2,3-dihydrobenzofuran hydrochloride (*7D*).

A mixture of 30 g of *7D*, 150 ml of tetrahydrofuran and 24.5 g of ethylidiisopropylamine was chilled to 5°C while 5.1 g of methyl chloroformate was added drop-by-drop over a period of 10 minutes. The resulting mixture was stirred for 30 minutes at 5°C, then warmed to and held at 60°C for 10 minutes. The volatile materials were evaporated. The residue was taken up in 300 ml of water and extracted with ether. The extract was dried ($MgSO_4$) and the solvent was partially evaporated. The residue was chilled and filtered to give methyl 3-(2,3-dihydrobenzofuran-7-yl)hydrazinecarboxylate (*7E*), as an off-white solid, m.p.: 110—112°C.

A solution of 3.5 g of *5* in 25 ml of benzene was added drop-by-drop over a period of 5 minutes at room temperature to a stirred solution of 5 g of phosgene in 75 ml of benzene. The mixture was refluxed for one hour, the solvent was evaporated, and the residue was crystallized from methanol to give (7), as a colourless solid, m.p.: 110—112°C.

Example 8

3-(2,3-dihydrobenzofuran-7-yl)-5-methoxy-1,3,4-oxadiazol-2(3H)-one (*8*)

0.123 g of *7* was mixed with 2 ml of methanol and 0.1 ml of triethylamine and the mixture was allowed to stand overnight. The mixture was diluted with ether, washed with dilute hydrochloric acid, and dried ($MgSO_4$), and the solvent was evaporated. The residue was purified by thin layer chromatography to give *8* as a cream-coloured solid; m.p. 108—109°C.

Activity of compounds of this invention with respect to insect and acarine pests were determined by using standarized test methods to measure the toxicity of the compounds as follows:

1. Houseflies (*Musca domestica* (Linne)) were tested by placing 50 4- to 5- day old houseflies into a spray cage and spraying with 0.6 ml of a solution of test compound. After spraying, the flies were anesthetized with $CO_2$ and transferred to a recovery cage containing a milk pad for food.

The cages were held for 18—20 hours after which mortality counts were made. Both dead and moribund flies were counted. The tests were conducted employing seveal dosage rates for each test compound.

2. Pea aphids (*Acyrthosiphon pisum* (Harris)) were tested by placing about 100 aphids on broad bean plants. The plants were sprayed with dilutions of an acetone solution of the test compound in water containing an emulsifier and held in containers under laboratory conditions for 18 to 20 hours, at which time the living aphids in the containers were counted. The tests were conducted employing several different dosage rates for each test compound.

3. Adult female two-spotted spider mites (*Tetranychus urticae* (Koch)) were tested by placing 50—75 mites on the bottom side of leaves of pinto bean plants. The leaves were sprayed with dilutions of an acetone solution of the test compound in water containing an emulsifier and kept under laboratory conditions for about 20 hours, at which time mortality counts were made. The tests were conducted employing several different dosage rates for each test compound.

4. Mosquito larvae (*Anophelus albimanus* (Weide)) were tested by placing 10 living and active mosquito larvae in a jar containing a 0.1 ml aliquot of 1% acetone solution of the test compound thoroughly mixed with 100 ml of distilled water. After 18—22 hours, mortality counts were taken. Both dead and moribund larvae were counted as dead. Larvae which did not swim after being prodded with a needle were considered moribund. The tests were conducted employing several different dosage rates for each compound.

5. Corn earworm larvae (*Heliothis zea* (Boddie)) were tested by spraying a broad bean plant with dilutions of an acetone solution of the test compound in water containing an emulsifier. Immediately after spraying, 5 larvae were transferred to the plant and held for 44—46 hours, at which time the dead

and moribund larvae were counted. The tests were conducted employing several different dosage rates for each test compound.

In the case of each species of insect, the concentration of the test compound in the formulation required to kill fifty percent of the test insects — i.e., the $LC_{50}$ dosage — was determined. The results are set out in Table I, $LC_{50}$ figures being expressed in parts per million.

TABLE I

| Compound | $LC_{50}$ (ppm) | | | | |
|---|---|---|---|---|---|
| | Housefly | Pea Aphid | Corn Earworm | Spidermite | Mosquito larvae |
| 1 | > 0.5 | 0.00008 | 0.043 | > 0.02 | 0.016 |
| 2 | > 0.5 | 0.0003 | 0.1 | > 0.2 | > 0.1 |
| 4 | 0.005 | 0.00003 | 0.0218 | 0.03 | 0.006 |
| 5 | 0.03 | 0.00009 | 0.1 | 0.2 | > 0.1 |
| 6 | 0.019 | 0.00015 | 0.078 | 0.031 | > 0.1 |
| 8 | 0.05 | 0.0006 | 0.023 | – | – |

## Claims

1. A 7-substituted 2,3-dihydrobenzofuran of general formula

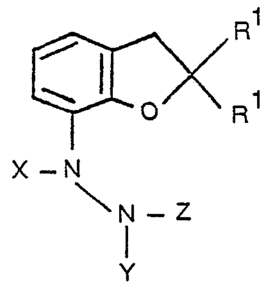

(I)

wherein each $R^1$ represents a hydrogen atom or a methyl group: X, Y and Z together represent a bridging group —CO—O—C(OR)=, or X is a chlorocarbonyl group, Y is hydrogen and Z is a carboxylate ester group —COOR, or X and Y both hydrogen and Z is a carboxylate ester group —COOR or a sulfonic acid group —$SO_3H$, or a salt thereof; and R represents a methyl, ethyl or propynyl group.

2. A 7-substituted 2,3-dihydrobenzofuran according to Claim 1 wherein when each $R^1$ represents a hydrogen atom, X, Y and Z together represent a bridging group —CO—O—C($OCH_3$)=, and when each $R^1$ represents a methyl group, X, Y and Z together represent a bridging group —CO—O—C(OR)= wherein R represents a methyl, ethyl or propynyl group or X is a chlorocarbonyl group, Y is hydrogen and Z is a carboxymethyl or carboxyethyl group.

3. A 7-substituted 2,3-dihydrobenzofuran of formula

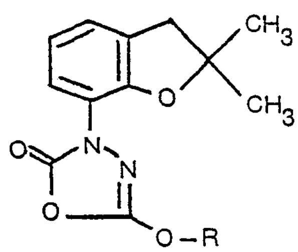

wherein R is methyl, ethyl or propynyl.

4. A 7-substituted 2,3-dihydrobenzofuran of formula

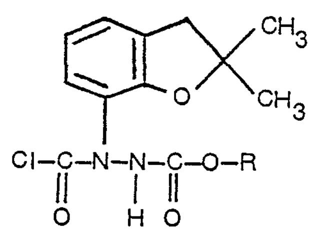

(III)

wherein R is methyl or ethyl.

5. 3-(2,3-Dichlorobenzofuran-7-yl)-5-methoxy-1,3,4-oxodiazol-2(3H)-one.

6. A process for preparing a 7-substituted 2,3-dihydrobenzofuran of formula I as defined in any of Claims 1 to 5 wherein X, Y and Z together represent a bridging group —CO—O—C(OR)=, or X is a chlorocarbonyl group, Y is hydrogen and Z is a carboxylate ester group —COOR, and R represents a methyl, ethyl or propynyl group, which process comprises reacting an ester of formula

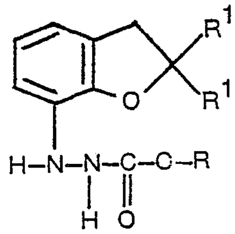

(IV)

wherein R and $R^1$ are as defined in Claim 1 with phosgene in an inert solvent, followed where necessary by treating the resulting compound of formula I wherein X is a chlorocarbonyl group, Y is hydrogen and Z is carboxylate ester group —COOR with a base to produce a compound of formula I wherein X, Y and Z together represent a bridging group —CO—O—C(OR)=.

7. A pesticidal composition comprising a 7-substituted 2,3-dihydrobenzofuran of formula I as defined in any one of claims 2 to 5 in association with an inert carrier therefor.

8. A method of combating pests at a locus which comprises applying to the locus a 7-substituted 2,3-dihydrobenzofuran of formula I as defined in any one of Claims 2 to 5 or a composition according to Claim 7.

**Patentansprüche**

1. In 7-Stellung substituiertes 2,3-Dihydrobenzofuran der allgemeinen Formel

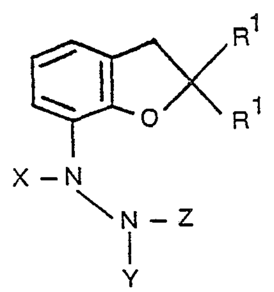

(I)

in der jedes $R^1$ ein Wasserstoffatom oder eine Methylgruppe bedeutet, X, Y und Z zusammen eine Brückengruppe —CO—O—C(OR)= bedeuten oder X eine Chlorcarbonylgruppe, Y ein Wasserstoffatom und Z eine Carboxylatestergruppe —COOR ist oder X und Y jeweils Wasserstoffatome sind und Z eine Carboxylatestergruppe —COOR oder eine Sulfonsäuregruppe —$SO_3H$ oder ein Salz davon ist und R eine Methyl-, Ethyl- oder Propinylgruppe bedeutet.

2. In 7-Stellung substituiertes 2,3-Dihydrobenzofuran nach Anspruch 1, wobei, wenn jedes $R^1$ ein Wasserstoffatom bedeutet, X, Y und Z zusammen eine Brückengruppe —CO—O—C($OCH_3$)= bedeuten und wenn jedes $R^1$ eine Methylgruppe ist, X, Y und Z zusammen eine Brückengruppe —CO—O—C(OR)= bedeuten, wobei R eine Methyl-, Ethyl- oder Propinylgruppe ist oder X eine Chlorcarbonylgruppe, Y ein Wasserstoffatom und Z eine Carboxymethyl- oder Carboxyethylgruppe bedeuten.

3. In 7-Stellung substituiertes 2,3-Dihydrobenzofuran der Formel

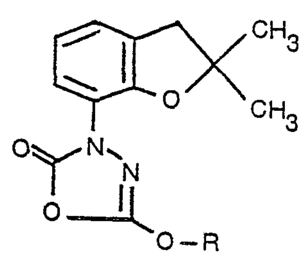

in der R eine Methyl-, Ethyl- oder Propinylgruppe ist.

4. In 7-Stellung substituiertes 2,3-Dihydrobenzofuran der Formel

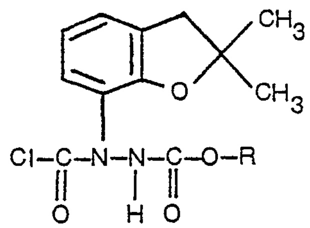

(III)

in der R eine Methyl- oder Ethylgruppe ist.

5. 3-(2,3-Dichlorbenzofuran-7-yl)-5-methoxy-1,3,4 -oxadiazol-2(3H)-on.

6. Verfahren zur Herstellung eines in 7-Stellung substituierten 2,3-Dihydrobenzofurans der Formel I, wie in einem der Ansprüche 1 bis 5 definiert, wobei X, Y und Z zusammen eine Brückengruppe —CO—O—C(OR)= oder X eine Chlorcarbonylgruppe, Y ein Wasserstoffatom und Z eine Carboxylatestergruppe —COOR bedeuten und R eine Methyl-, Ethyl- oder Propinylgruppe ist, umfassend die Umsetzung eines Esters der Formel

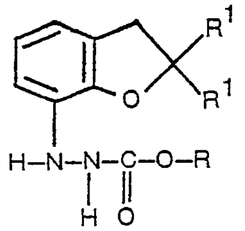

(IV)

in der R und $R^1$ die in Anspruch 1 angegebene Bedeutung haben, mit Phosgen in einem inerten Lösungsmittel und anschließend, wenn erforderlich, Behandlung der erhaltenen Verbindung der Formel I, in der X eine Chlorcarbonylgruppe, Y ein Wasserstoffatom und Z eine Carboxylatestergruppe —COOR ist, mit einer Base unter Bildung einer Verbindung der Formel I, in der X, Y und Z zusammen eine Brückengruppe —CO—O—C(OR)= bedeuten.

7. Pestizides Mittel, enthaltend ein in 7-Stellung substituiertes 2,3-Dihydrobenzofuran der Formel I, wie in einen der Ansprüche 2 bis 5 definiert, zusammen mit einem inerten Träger dafür.

8. Verfahren zur Bekämpfung von Schädlingen an einer Stelle, umfassend das Aufbringen eines in 7-Stellung substituierten 2,3-Dihydrobenzofurans der Formel I, wie in einem der Ansprüche 2 bis 5 definiert, oder eines Mittels nach Anspruch 7 auf die Stelle.

**Revendications**

1. Un 2,3-dihydrobenzofuranne substitué en position 7 de la formule générale

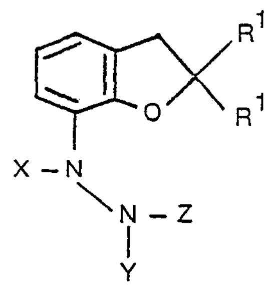

(I)

dans laquelle chaque $R^1$ représente un atome d'hydrogène ou un groupe méthyle; X, Y et Z représentent ensemble un groupe formant pont —CO—O—C(OR)=, ou X est un groupe chlorocarbonyle, Y est de l'hydrogène et Z est un groupe ester carboxylique —COOR, ou X et Y sont tous deux de l'hydrogène et Z est un groupe ester carboxylique —COOR ou un groupe acide sulfonique —$SO_3H$, ou un sel correspondant; et R représente un groupe méthyle, éthyle ou propynyle.

2. Un 2,3-dihydrobenzofuranne substitué en position 7 selon la revendication 1, dans lequel chaque $R^1$ représente un atome d'hydrogène, X, Y et Z représentent ensemble un groupe formant pont

—CO—O—C($OCH_3$)=, et quand chaque $R^1$ représente un groupe méthyle, X, Y et Z représentent ensemble un groupe formant pont —CO—O—C(OR)= où R représente un groupe méthyle, éthyle ou propynyle ou X est un groupe chlorocarbonyle, Y est de l'hydrogène et Z est un groupe carboxyméthyle ou carboxyéthyle.

3. Un 2,3-dihydrobenzofuranne substitué en position 7 de formule

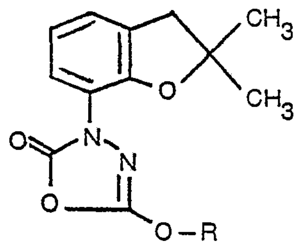

dans laquelle R est un groupe méthyle, éthyle ou propynyle.

4. Un 2,3-dihydrobenzofuran substitué en position 7 de formule

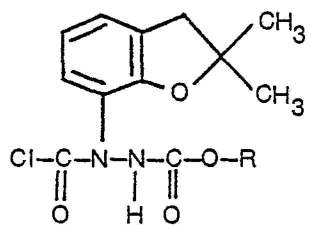

(III)

dans laquelle R est un groupe méthyle ou éthyle.

5. 3-(2,3-dichlorobenzofuran-7-yl)-5-méthoxy-1,3,4-oxadiazol-2(3H)-one.

6. Un procédé pour la préparation d'un 2,3-dihydrobenzofuranne substitué en position 7 de formule I tel que défini dans l'une quelconque des revendications 1 à 5, où X, Y et Z représentent ensemble un groupe formant pont —CO—O—C(OR)=, ou X est un groupe chlorocarbonyle, Y est de l'hydrogène et Z est un groupe ester carboxylique —COOR, et R représente un groupe méthyle, éthyle ou propynyle, selon lequel on fait réagir un ester de formule

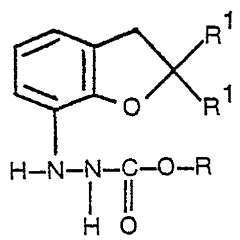

(IV)

dans laquelle R et $R^1$ sont tels que définis dans la revendication 1 avec du phosgène dans un solvant inerte, cela étant suivi quand c'est nécessaire d'un traitement du composé résultant de formule I dans lequel X est un groupe chlorocarbonyle, Y est de l'hydrogène et Z est un groupe ester carboxylique —COOR par une base de façon à produire un composé de formule I dans lequel X, Y et Z représentent ensemble un groupe formant pont —CO—O—C(OR)=.

7. Une composition pesticide comprenant un 2,3-dihydrobenzofuranne substitué en position 7 de formule I comme défini dans l'une quelconque des revendications 2 à 5 en association avec un véhicule inerte approprié.

8. Une méthode de lutte contre des organismes nuisibles en un lieu, qui comprend l'application au lieu concerné d'un 2,3-dihydrobenzofuranne substitué en position 7 de formule I comme défini dans l'une quelconque des revendications 2 à 5 ou d'une composition selon la revendication 7.